# EUROPEAN PATENT APPLICATION

(11) **EP 4 807 668 A1**
(43) Date of publication of application: **16.09.2026**
(21) Application number: 25162541.4
(22) Date of filing: 10.03.2025
(51) Int. Cl.: G06T 7/00, G06T 7/136, G06T 7/62, A61B 5/107, A61B 8/00

(54) **SYSTEM AND METHOD OF DETERMINING THICKNESS OF ORGAN WALL USING CALIPERS IN ULTRASOUND IMAGE**

(71) Applicant: Koninklijke Philips N.V., 5656 AE Eindhoven (NL)
(72) Inventor: SAI PHANI KUMAR, Malladi, Eindhoven (NL); VAJINEPALLI, Pallavi, Eindhoven (NL); XIE, Hua, 5656AG Eindhoven (NL); ERRICO, Claudia, Eindhoven (NL); BRUNELLE, Elizabeth, Eindhoven (NL); SADEGHI, Seyedali, Eindhoven (NL); BAI, Xianghui, Eindhoven (NL)
(74) Representative: Philips Intellectual Property & Standards

(57) **Abstract**

A method of determining thickness of an organ wall includes inputting an ultrasound image of an organ to a trained machine learning model configured to estimate a thickness measurement location on the organ wall (S1212); extracting intermediate feature maps from the machine learning model showing different portions of the organ wall as highlighted salient regions (S1213); linearly combining the extracted intermediate feature maps to provide a combined feature map (S1214); performing adaptive intensity thresholding on pixel intensities of pixels in the combined feature map using a threshold that preserves the highlighted salient regions to identify candidate regions of the wall suitable for thickness measurements (S1215); determining locations of calipers corresponding to the candidate regions for determining wall thicknesses (S1216); launching sets of calipers at the locations (S1217); selecting a candidate region based on the launched sets of calipers (S1218); and optionally computing a thickness value of the organ wall using the launched calipers in the selected candidate region (S1219).

## Description

### FIELD OF THE INVENTION

The invention relates to the field of ultrasound imaging, and more specifically to accurately and consistently determining caliper placement locations for measuring wall thickness of organs using ultrasound imaging.

### BACKGROUND OF THE INVENTION

Abdominal anatomy measurements made using ultrasound imaging scans are primarily used for diagnosing anatomy-related abnormalities, such as liver parenchyma, kidney stones, and gallbladder pathology. In this context, patient-specific and clinically relevant factors that influence accuracy and reliability of the measurements include patient position, pathological variations, patient habitus, and operator skill, for example.

Measuring wall thickness of a gallbladder, in particular, is crucial for evaluating gallbladder health and identifying potential abnormalities, such as cholecystitis (inflammation). The gallbladder wall thickness measurements help in assessing severity of any gallbladder pathology. For example, studies suggest that a gallbladder wall thickness of 3 mm is the upper limit of normal thickness values, and that a gallbladder wall thickness substantially larger than that suggests pathology. The gallbladder wall thickness measurements using ultrasound imaging is typically made on the anterior wall of the gallbladder, i.e., between the liver and gallbladder lumen, from the most inner boundary to the most outer boundary of the gallbladder wall. To avoid error, the measurement should be perpendicular to the gallbladder wall at the thickest part in case of focal thickening.

However, the measurement process may be time-consuming and is fraught with potential for human error. Because the gallbladder wall is quite thin, a minor deviation in its thickness measurement contributes to a significant portion of error. Also, it is difficult to perform precise measurements of the thin gallbladder walls that conform to clinical principles. Conventional techniques typically focus on manual measurements using retrospective data and/or contrast-enhanced ultrasonography, unlike standard B-mode imaging style. Typically, transverse or sagittal views of the gallbladder may be considered for wall measurements, depending on region (e.g., transverse views are preferred in the United States and India, while sagittal views are preferred in China).

In addition, clinician subjectivity influences the consistency of gallbladder wall measurements and resulting patient outcomes. Example sources of clinician subjectivity that may affect gallbladder wall thickness measurements include measurement techniques, image interpretation, and measurement consistency. Measurement techniques include variability in measurement methods, where different clinicians may use slightly different techniques or approaches for measuring the gallbladder wall thickness. For example, some clinicians may measure at the thickest part of the gallbladder wall, while others may measure at different locations regardless of relative thicknesses. Also, interpretation of boundaries of the gallbladder wall in an ultrasound image may vary since the boundaries are sometimes difficult to define precisely, leading to variations as to where measurements are taken. Image interpretation is subject to subjective visual perception and quality of the ultrasound images. Image quality depends on factors such as resolution and contrast, variations of which may affect how clearly the gallbladder wall is visualized and measured. Also, clinicians may perceive and delineate structures differently based on respective experiences and training. Measurement consistency is likewise affected by intra-observer variability, where the same clinician obtains slightly different measurements at different times due to factors like changes in technique or patient positioning, and inter-observer variability, where different clinicians produce different measurements for the same patient, e.g., based on different techniques, experience and skill levels, leading to inconsistent results.

### SUMMARY OF THE INVENTION

The invention is defined by the independent claims. Advantageous embodiments are provided in the dependent claims.

In accordance with an aspect of the present invention, a method for determining caliper placements for measuring wall thickness of an organ is provided. The method comprises inputting an ultrasound image of the organ to a trained machine learning model configured to estimate a thickness measurement location on an organ wall of the organ; extracting intermediate feature maps from the machine learning model, wherein the intermediate feature maps show different portions of the organ wall, respectively, as highlighted salient regions; linearly combining the extracted multiple intermediate feature maps to provide a combined feature map including the highlighted salient regions of the organ wall; performing adaptive intensity thresholding on pixel intensities of pixels in the combined feature map using a threshold that preserves the highlighted salient regions of the organ wall to identify candidate regions of the organ wall suitable for thickness measurements; determining locations of calipers in sets of calipers on the organ wall in the candidate regions for determining wall thicknesses of the organ wall, respectively; launching calipers in the sets of calipers on the organ wall in the plurality of candidate regions at the determined locations, respectively; and selecting a candidate region of the multiple candidate regions for determining the wall thickness of the organ wall based on the launched calipers in the sets of calipers. The present invention enables accurate and consistent determination of caliper placements for measuring the wall thickness of an organ using ultrasound imaging, reducing human error and improving measurement reliability. In a preferred embodiment, the method further comprises computing a thickness value of the organ wall at the selected candidate region using the launched calipers. In this way, the organ wall thickness may be measured with high accuracy.

In some embodiments, performing the adaptive intensity thresholding may comprise statistically examining the combined feature map to determine the threshold that preserves the highlighted salient regions of the organ wall; applying the threshold to the pixel intensities of the pixels in the combined feature map; and preserving pixels having pixel intensities exceeding the threshold, wherein the preserved pixels correspond to the highlighted salient regions, which are identified as the plurality of candidate regions, respectively. The preservation of highlighted salient regions of the organ wall by applying adaptive intensity thresholding is advantageous to ensure that only the most relevant regions are considered for thickness measurements.

In some embodiments, determining the locations of the calipers in the sets of calipers in the plurality of candidate regions for determining wall thicknesses of the organ may comprise performing skeletonization on each candidate region of the plurality of candidate regions to determine a thickest part of the organ wall in the candidate region; and performing ellipse-fitting to each candidate region of the plurality of candidate regions, wherein locations of calipers in a set of calipers in each candidate region is at the thickest part of the organ wall in the candidate region and has a perpendicular orientation to an orientation of a major axis of the fitted ellipse. The identification of the thickest part of the organ wall in each candidate region through skeletonization and ellipse-fitting may possibly lead to more accurate caliper placements. In such embodiments, launching the calipers may preferably include placing the calipers of each set of calipers on wall boundaries of the organ wall connected by a line perpendicular to the organ wall based on the fitted ellipse. In this way, the placement of calipers on the wall boundaries can be more precise, ensuring more accurate thickness measurements.

In a preferred embodiment, the method further comprises initially training the machine learning model using a plurality of training ultrasound images showing organ anatomies, respectively, wherein each training ultrasound image of the plurality of training ultrasound images is annotated to identify locations of calipers in a set of calipers on an organ wall for measuring thickness of the organ wall. More preferably, the training may advantageously comprise initializing the machine learning model with a default set of weights and a default set of hyper-parameters. The initial training of the machine learning model using annotated training ultrasound images may advantageously improve the model's ability to accurately determine caliper placements. Moreover, the initialization with default weights and hyper-parameters may further enhance the model's training efficiency and accuracy.

In some embodiments, the machine learning model may preferably be a deep neural network comprising at least one initial layer, at least one middle layer, and at least one end layer, and wherein the plurality of intermediate feature maps are extracted from the at least one end layer of the deep neural network. In this way, more detailed and relevant information can be provided for determining caliper placements.

More preferably, the deep neural network may comprise a You Only Look Once (Yolo), version 8, pose estimation network. In such case, the at least one initial layer may preferably extract low-level cues from the training ultrasound images, and provide basic understanding about structure and boundaries within the input ultrasound image; the at least one middle layer may preferably start inferring task-specific context regarding anatomy specific details, wherein learning information about the organ walls is a given task, and the at least one end layer may preferably extract high-level cues involving complex interpretations, wherein the organ walls are targets causing the at least one end layer to highlight the organ walls by projecting them predominantly. Additionally or alternatively, the at least one end layer may advantageously comprise a plurality of end layers, and wherein the plurality of intermediate feature maps may be extracted from a selected end layer of the plurality of end layers that activates the most with regard to the number of the highlighted salient regions of the organ wall and best contrast or brightness in the plurality of intermediate feature maps. The use of a Yolo pose estimation network has proven to be very efficient for this particular problem and capable of identifying caliper placements with high accuracy. The extraction of low-level, task-specific, and high-level cues by different layers of the deep neural network, may further improve the model's ability to accurately identify caliper placements. Moreover, the extraction of intermediate feature maps from the end layer that activates the most may advantageously ensure that the most relevant information is used for determining caliper placements.

In some embodiments, the method may further comprise displaying the locations of the calipers in the launched sets of calipers as key points within bounding boxes surrounding the sets of calipers in the plurality of candidate regions, wherein the candidate region of the plurality of candidate regions is selected based on the displayed locations of the calipers within the bounding boxes. This provides particularly useful visual aids to the user for the selection of the most suitable candidate region for thickness measurements.

In a preferred embodiment, the organ may be a gallbladder. The specific anatomical structure and morphology of the gallbladder make the methods described above particularly suitable for this organ.

In accordance with another aspect of the present invention, a system for determining caliper placements for measuring wall thickness of an organ is provided. The system comprises a display, at least one processing unit coupled to the display, and at least one non-transitory memory. The at least one non-transitory memory stores instructions which, when executed by the at least one processing unit, cause the at least one processing unit to perform any of the methods described above.

In another representative embodiment, a system for determining caliper placements for measuring wall thickness of a gallbladder includes a display, at least one processing unit coupled to the display, and at least one non-transitory memory. The memory stores instructions which, when executed by the at least one processing unit, cause the at least one processing unit to input an ultrasound image of the gallbladder to a trained machine learning model configured to estimate a thickness measurement location on a gallbladder wall of the gallbladder; extract multiple intermediate feature maps from the machine learning model, wherein the multiple intermediate feature maps show different portions of the gallbladder wall, respectively, as highlighted salient regions; linearly combine the extracted multiple intermediate feature maps to provide a combined feature map including the highlighted salient regions of the gallbladder wall; perform adaptive intensity thresholding on pixel intensities of pixels in the combined feature map using a threshold that preserves the highlighted salient regions of the gallbladder wall to identify multiple candidate regions of the gallbladder wall suitable for thickness measurements; determine locations of calipers in sets of calipers in the multiple candidate regions for determining wall thicknesses of the gallbladder wall, respectively; launch calipers in the sets of calipers on the gallbladder wall in the multiple candidate regions at the determined locations, respectively; and determine a selection of a candidate region of the multiple candidate regions for determining the wall thickness of the gallbladder wall based on the launched calipers in the sets of calipers. The execution of the instructions may further cause the at least one processing unit to compute a thickness value of the gallbladder wall at the selected candidate region using the launched calipers in the set of calipers.

The execution of the instructions may further cause the at least one processing unit to perform the adaptive intensity thresholding by statistically examining the combined feature map to determine the threshold that preserves the highlighted salient regions of the gallbladder wall; applying the threshold to the pixel intensities of the pixels in the combined feature map; and preserving pixels having pixel intensities exceeding the threshold, wherein the preserved pixels correspond to the highlighted salient regions, which are identified as the plurality of candidate regions, respectively.

The execution of the instructions may further cause the at least one processing unit to determine the locations of the calipers in the sets of calipers by performing skeletonization on each candidate region of the plurality of candidate regions to determine a thickest part of the gallbladder wall in the candidate region; and performing ellipse-fitting to each candidate region of the plurality of candidate regions, wherein locations of calipers in a set of calipers in each candidate region is at the thickest part of the gallbladder wall in the candidate region and has a perpendicular orientation to an orientation of a major axis of the fitted ellipse. In such case, the execution of the instructions may preferably cause the at least one processing unit to launch the calipers in the sets of calipers in the plurality of candidate regions by placing the calipers of each set of calipers on wall boundaries of the gallbladder wall connected by a line perpendicular to the gallbladder wall based on the fitted ellipse.

The execution of the instructions may further cause the at least one processing unit to initially train the machine learning model using a plurality of training ultrasound images showing gallbladder anatomies, respectively, wherein each training ultrasound image of the plurality of training ultrasound images is annotated to identify locations of calipers in a set of calipers on a gallbladder wall for measuring thickness of the gallbladder wall.

The plurality of intermediate feature maps may preferably be extracted from a selected end layer of a plurality of end layers in the machine learning model, wherein the selected end layer is an end layer of the plurality of end layers that activates the most with regard to the number of the highlighted salient regions of the gallbladder wall and best contrast or brightness in the plurality of intermediate feature maps.

The execution of the instructions may further cause the at least one processing unit to display the locations of the launched calipers in the sets of calipers as key points within bounding boxes surrounding the sets of calipers corresponding to the plurality of candidate regions, wherein the selection of the candidate region of the plurality of candidate regions is based on the displayed locations of the calipers within the bounding boxes.

In accordance with yet another aspect of the present invention, there is also provided a non-transitory computer readable medium storing instructions which, when executed by at least one processing unit, cause the at least one processing unit to perform any of the methods described above.

In another representative embodiment, a non-transitory computer readable medium stores instructions which, when executed by at least one processing unit, cause the at least one processing unit to input an ultrasound image of the gallbladder to a trained machine learning model configured to estimate a thickness measurement location on a gallbladder wall of the gallbladder; extract multiple intermediate feature maps from the machine learning model, wherein the multiple intermediate feature maps show different portions of the gallbladder wall, respectively, as highlighted salient regions; linearly combine the extracted multiple intermediate feature maps to provide a combined feature map including the highlighted salient regions of the gallbladder wall; perform adaptive intensity thresholding on pixel intensities of pixels in the combined feature map using a threshold that preserves the highlighted salient regions of the gallbladder wall to identify multiple candidate regions of the gallbladder wall suitable for thickness measurements; determine locations of calipers in sets of calipers in the multiple candidate regions for determining wall thicknesses of the gallbladder wall, respectively; launch calipers in the sets of calipers on the gallbladder wall in the plurality of candidate regions at the determined locations, respectively; and determine a selection of a candidate region of the multiple candidate regions for determining wall thickness of the gallbladder wall based on the launched calipers in the sets of calipers.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is an ultrasound image of a sagittal view of a gallbladder with different caliper locations for measuring thickness of the gallbladder wall.
Fig. 2 is a simplified block diagram of a system for determining caliper placements for measuring wall thickness of a gallbladder, according to a representative embodiment.
Fig. 3 is shows illustrative images showing feature maps at different layers generated by the machine learning model based on a sagittal view of the gallbladder, according to a representative embodiment.
Fig. 4 shows a sampling of the intermediate feature maps extracted from one of the end layers generated by the machine learning model combined into a combined feature map, according to a representative embodiment.
Fig. 5 shows results of adaptive dynamic thresholding performed on the combined feature map to provide candidate regions, according to a representative embodiment.
Fig. 6 shows the skeletonization process performed on the candidate regions identified in the thresholding feature map to determine thickest parts of the candidate regions, according to a representative embodiment.
Fig. 7 shows an ellipse-fitting process performed on the candidate regions identified in the skeletonized feature map to determine orientations of the candidate regions, according to a representative embodiment.
Fig. 8 shows a process of determining caliper placements in the candidate regions identified in the skeletonized feature map, according to a representative embodiment.
Fig. 9 shows placements of launched calipers in the caliper placements feature map and displayed in the ultrasound image, according to a representative embodiment.
Fig. 10 shows a selected caliper placements displayed in the ultrasound image, according to a representative embodiment.
Fig. 11A shows an illustrative ultrasound image of a transverse view of a gallbladder for determining caliper placements, according to a representative embodiment.
Fig. 11B caliper placements on the gallbladder wall displayed in the ultrasound image, according to a representative embodiment.
Fig. 11C shows a selected caliper placements on the gallbladder wall displayed in the ultrasound image, according to a representative embodiment.
Fig. 12 is a flow diagram of a method of determining caliper placements for measuring wall thickness of a gallbladder, according to a representative embodiment.

### DETAILED DESCRIPTION OF EMBODIMENTS

Any of the steps described in relation to examples and/or training described below can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. As an example, a computer system performs logical processing based on digital signals received via an analogue-to-digital converter.

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

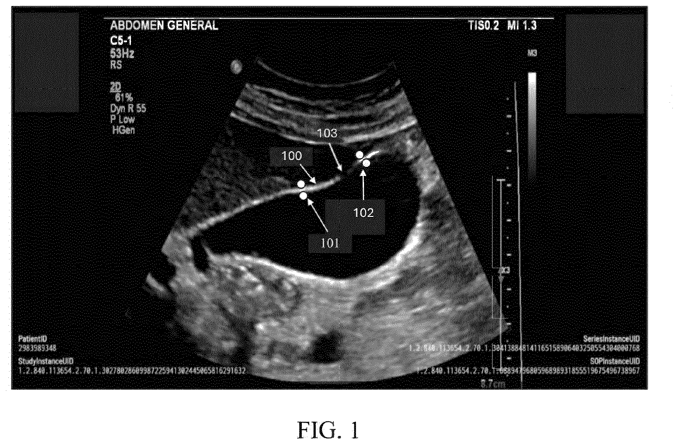

As mentioned above, different users (e.g., physicians, sonographers and other clinicians), may measure gallbladder wall thickness at different locations on the gallbladder wall based on individual interpretations of the ultrasound images. For instance, Fig. 1 is an ultrasound image showing a sagittal view of a gallbladder with calipers at first location 101 and second location 102 for measuring thickness of gallbladder wall 100 by two different users. The first and second locations 101 and 102 correspond to two completely different portions of the gallbladder wall, separated at a region 103, which is a discontinuity in the gallbladder wall from localized damage or changes in the wall structure. Even though both locations may result in roughly the same thickness measurement, varying perceptions and experience levels result in the measurements being taken at the different locations. Further, depending on the region of interest, the actual thickness values may vary, which may not provide a unified measurement. Also, some users may measure the blurry portion of the gallbladder wall boundary as an approximation. Such measurements may be difficult for other users to interpret, and may thus provoke additional manual measurements leading to redundant tasks demanding more time.

While users may generally accept each other's measuring calipers, reaching consensus on measurement styles can be quite challenging. Given that generalizing these variations is a complex task for humans, it is equally challenging for a machine learning model to learn these variations from a dataset with only a single set of wall caliper annotations corresponding to an image. Such human inconsistencies may be mitigated using a robust system discussed below that standardizes the measurement process and incorporates a wide range of clinical styles and domain knowledge. Such a system may provide customized outputs per requirements of the user, as well as present options to the user as a set of possible candidates for gallbladder wall thickness measurements abiding to clinical guidelines and data quality standards.

Generally, the various embodiments described herein provide a system and method for determining a location on an organ wall to place calipers for determining thickness of the organ wall using a hybrid technique that combines a machine learning model with a customized algorithm the extracts intermediate feature maps from the machine learning model, which may generate the intermediate feature maps at inference, to identify a set of possible candidate regions for measuring the organ wall. The machine learning model may be a deep neural network, for example, trained on an image dataset where annotations are represented by a set of wall calipers, a common structure used for training pose estimation deep neural networks. The representations capture organ wall portions with similar echogenicity, visibility and clarity, ensuring that the identified organ wall portions are good for measurement both by user and the automated system. Ultimately, the user chooses a candidate region from the proposed set of candidate regions that is most suitable, according to his/her interest. The set of calipers corresponding to the selected candidate region may then be launched on the selected organ wall portion boundary, and thickness value may be measured using the launched set of calipers.

In the following embodiments, determining placements of calipers for measuring wall thickness of an organ is discussed with reference to a gallbladder for purposes of illustration. It is understood, however, that the embodiments may apply equally to any organ in the body of a subject that requires a wall thickness measurement in an ultrasound imaging examination using calipers. Examples of organs requiring measurement of wall thickness include the uterus, the stomach and the bladder of the subject, in addition to the gallbladder.

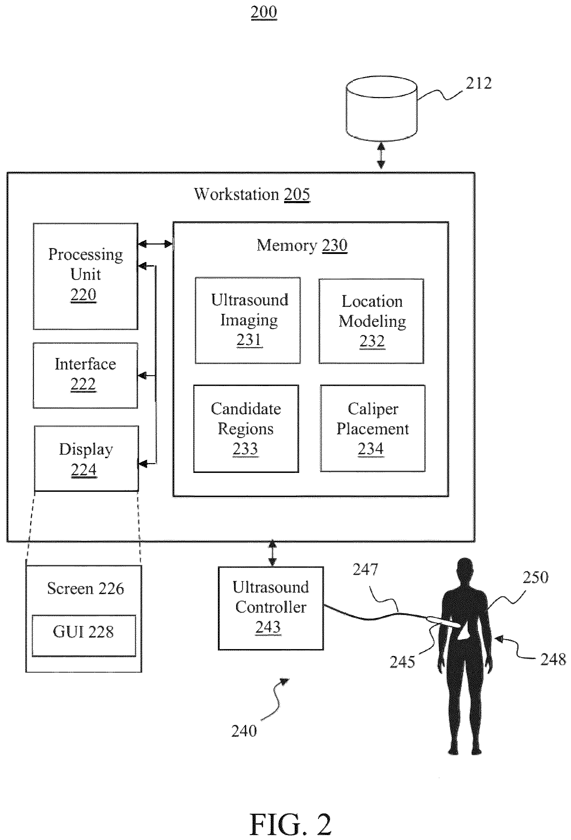

Fig. 2 is a simplified block diagram of a system for determining caliper placements for measuring wall thickness of a gallbladder, according to a representative embodiment.

Referring to Fig. 2, system 200 includes a workstation 205 for implementing and/or managing the processes described herein with regard to determining thickness of a gallbladder 248 in a subject 250 (patient) using ultrasound images from an ultrasound imaging system 240. The workstation 205 includes one or more processors indicated by processing unit 220, one or more memories indicated by memory 230, a user interface 222 and a display 224. The processing unit 220 communicates with the ultrasound imaging system 240 through an imaging interface (not shown). The ultrasound imaging system 240 includes an ultrasound controller 243 and an ultrasound probe 245 operable by an operator to obtain ultrasound images of the gallbladder 248 of the subject 250. The ultrasound probe 245 may be manipulated manually by the ultrasound operator, automatically by a robot under control of a robot controller (not shown), or a combination of both.

The ultrasound probe 245 may include a 2D matrix array of transducer elements, capable of scanning in two or three dimensions, for example, for emitting ultrasound waves into the body of the subject 250 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array may be coupled to a microbeamformer in the ultrasound probe 245, which controls transmission and reception of signals by the transducer elements.

The ultrasound probe 245 is connected to the ultrasound controller 243 via a probe cable 247. The ultrasound controller 243 is configured to control the ultrasound imaging process, and includes known elements for performing ultrasound imaging, such as a transmit/receive (T/R) switch configured to switch between transmission and reception modes, and a main beamformer configured to provide final beamforming. One of the functions performed by the ultrasound controller 243 is the direction in which beams are steered and focused. For example, beams may be steered straight ahead from (orthogonal to) the transducer array of the ultrasound probe 245, or at different angles for a wider field of view. Generally, the transmitting of ultrasound signals and the receiving and processing of echo signals in response is known, and therefore additional detail in this regard is not included herein. In various embodiments, all or part of the functionality of the ultrasound controller 243 may be implemented by the processing unit 220.

The memory 230 stores instructions executable by the processing unit 220. When executed, the instructions cause the processing unit 220 to implement one or more processes for determining locations for thickness measurements of the gallbladder wall in ultrasound images acquired by the ultrasound imaging system 240. The ultrasound images may be provided from the ultrasound imaging system 240 in real-time or near real-time during the scanning procedure, or may be retrieved from storage following the scanning procedure. For purposes of illustration, the memory 230 is shown to include software modules, each of which includes the instructions, executable by the processing unit 220, corresponding to an associated capability of the system 200.

The processing unit 220 is representative of one or more processors or processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hard-wired logic circuits, or combinations thereof. Any processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 230 may include main memory and/or static memory, where such memories may communicate with each other and the processing unit 220 via one or more buses. The memory 230 may be implemented by any number, type and combination of random access memory (RAM) and read-only memory (ROM), for example, and may store various types of information, such as software algorithms, artificial intelligence (AI) machine learning models, and computer programs, all of which are executable by the processing unit 220. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 230 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 230 may store software instructions and/or computer readable code that enable performance of various functions. The memory 230 may be secure and/or encrypted, or unsecure and/or unencrypted.

The system 200 may also include a database 212 for storing information that may be used by the various software modules of the memory 230. For example, the database 212 may include image data from previously obtained ultrasound images of the subject 250 and/or of other similarly situated subjects. The stored image data may be used for training an AI machine learning model, such as a neural network model, for example, as discussed below. The database 212 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 212 comprises tangible storage mediums for storing data and executable software instructions and is non-transitory during the time data and software instructions are stored therein. The database 212 may be secure and/or encrypted, or unsecure and/or unencrypted. For purposes of illustration, the database 212 is shown as a separate storage medium, although it is understood that it may be combined with and/or included in the memory 130, without departing from the scope of the present teachings.

The processing unit 220 may include or have access to an artificial intelligence (AI) engine, which may be implemented as software that provides artificial intelligence (e.g., deep learning, neutral network models) and applies machine learning described herein. The AI engine may reside in any of various components in addition to or other than the processing unit 220, such as the memory 230, an external server, and/or the cloud, for example. When the AI engine is implemented in a cloud, such as at a data center, for example, the AI engine may be connected to the processing unit 220 via the internet or other communication network using one or more wired and/or wireless connection(s). In various embodiments, all or part of the process provided by machine learning model discussed below may be implemented by the AI engine, for example. The training and execution of the machine learning model cannot practically be performed in the human mind.

The user interface 222 is configured to provide information and data output by the processing unit 220, the memory 230 and/or the ultrasound imaging system 240 to the user and/or for receiving information and data input by the user. That is, the user interface 222 enables the user to enter data and to control or manipulate aspects of the processes described herein, and also enables the processing unit 220 to indicate the effects of the user's input, which may include control or manipulation of the ultrasound probe 245. All or a portion of the user interface 222 may be implemented by a graphical user interface (GUI), such as GUI 228 viewable on the display 224, discussed below. The user interface 222 may include one or more interface devices, such as a mouse, a keyboard, a trackball, a joystick, a microphone, a video camera, a touchpad, a touchscreen, voice or gesture recognition captured by a microphone or video camera, for example.

The display 224 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 224 includes a screen 226 for viewing ultrasound images of the subject 250, along with various features described herein to communicate to the user the degree of image degradation, if any, as well as the GUI 228 to enable the user to interact with the displayed images and features. In an embodiment, the ultrasound imaging system 240 may include a separate dedicated display for acquiring the ultrasound images, where dedicated display is also represented by the display 224.

Referring to the memory 230, the various modules store sets of data and instructions executable by the processing unit 220 to determine locations of calipers on a wall of the gallbladder 248 shown in an ultrasound image for purposes of determining a location for measuring thickness of the gall bladder wall in the ultrasound image.

The memory 230 includes ultrasound image module 231, which is configured to receive and process ultrasound images acquired by the ultrasound probe 245 of the ultrasound imaging system 240 to provide corresponding ultrasound image data. The ultrasound images are obtained from scanning the subject's abdomen using the ultrasound probe 245, as discussed above. The ultrasound images may be received in real-time or near real-time from the ultrasound imaging system 240, e.g., during a contemporaneous imaging session of the subject 250, and may be displayed on the display 224. The display of real-time images would enable the operator to visualize to some extent the anatomy of the subject 250 while performing sweeps using the ultrasound probe 245. Alternatively, or in addition, the ultrasound images may be previously acquired images, obtained during a previous imaging session of the subject 250 and retrieved from storage (e.g., database 212). The ultrasound image module 231 may also store data associated with the ultrasound images, such as time and date of image acquisition, identification of the ultrasound imaging system 240, and identification of the user operating the ultrasound imaging system 240 to acquire the ultrasound images.

Location modeling module 232 is configured to train and execute a machine learning model that automatically determines a measurement location for placement of a set of calipers on the wall of the gallbladder 248 for measuring thickness of the gallbladder wall. Notably, the measurement location for the set of calipers as estimated by the machine learning model is nominal, since it will not actually be used as the final location for placement of calipers, according to the embodiments herein. Rather, intermediate feature maps, generated by the machine learning model for determining the nominal measurement location, are extracted in order to determine multiple candidate regions for placing multiple sets of calipers for measuring gallbladder wall thickness, respectively, as discussed below.

Generally, the machine learning model provided by the location modeling module 232 receives the ultrasound image data from the ultrasound image module 231 as input, and provides the nominal locations of the calipers on the gallbladder wall as output. The machine learning model may be implemented as any suitable type of trainable machine learning model, such as a deep neural network (NN), a convolutional neural network (CNN), an artificial neural network (ANN), a vision transformer, or a U-net model, for example. In an embodiment, the machine learning model is a deep neural network, such as a You Only Look Once (Yolo) pose estimation network (e.g., version 8) or HRNet, for example. A deep neural network model, in particular, has large numbers of layers and parameters, which directly enable tasks such as classification and regression, for example, as would be apparent to one skilled in the art.

The machine learning model is previously trained in a supervised fashion using a training dataset including ultrasound image data from thousands of training ultrasound images of gallbladder anatomies, respectively. Of course, training the machine learning model for providing nominal locations of calipers on the walls of other organs would involve a training dataset including ultrasound image data from training ultrasound images of those other organs rather than the gallbladder. The training dataset for training the machine learning model may be stored in the database 212, for example. Each training ultrasound image is labelled (annotated) to identify two key points indicating locations of calipers in a set of calipers on a gallbladder wall of the depicted gallbladder used to measure thicknesses of the gallbladder wall. The key points are on the outer and inner boundaries of the gallbladder wall at the measurement location. The measurement location typically corresponds to the thickest portion of the gallbladder wall in the training ultrasound images, as discussed above. The labels may be added by experts in ultrasound imaging, such as physicians, radiologists and sonographers, for example, or may be labeled automatically through techniques such as ground truth automation, as would be apparent to one skilled in the art. The labeled training dataset may be referred to as ground truth data. The training may further include initializing the machine learning model with a default set of weights and a default set of hyper-parameters in order to set some initial weights for the connections between neurons across different layers. In an embodiment, weights are initialized based on the layers and activations. For example, Xavier initialization is used for sigmoid or tangent hyperbolic (tanh) activation functions and He initialization is used for rectified linear unit (ReLU) activation function. In an embodiment, the machine learning model may use key point detection loss (e.g., Euclidean norm) as the loss function, for example, during training.

The machine learning model is thus trained end-to-end using the training dataset to detect a location of a set of calipers on the gallbladder wall for measuring thickness. At inference, the trained machine learning model receives as input an ultrasound image of the subject 250 to begin the process of predicting caliper locations of a set of calipers on the gallbladder wall for measuring thickness. The trained machine learning model identifies a location on the gallbladder wall in the ultrasound image data most suitable for wall thickness evaluation, based on relative thicknesses of different locations and/or clarity of the gallbladder wall, for example. The most suitable location may be identified from multiple locations using non-maximum suppression, for example, which refines and removes duplicates. The trained machine learning model is further configured to determine key points on outer and inner boundaries of the identified portion of the gallbladder wall, and to launch the determined key points on the outer and inner boundaries of the identified portion of the gallbladder wall as respective locations of calipers in the set of calipers.

However, as mentioned above, the output of the machine learning model is not actually used. Rather, the trained machine learning model (e.g., deep neural network model) includes at least one initial layer, at least one middle layer, and at least one end layer. When the machine learning model is a Yolo pose estimation network (e.g., version 8), for example, the initial layers are referred to as backbone layers, the middle layers are referred to as neck layers, and the end layers are referred to as head layers. The embodiments described herein extract intermediate feature maps from one of the end layers, where the intermediate feature maps may be generated at inference by the machine learning model in order to estimate multiple possible caliper placements, as discussed below.

Candidate regions module 233 is configured to automatically identify candidate regions of the gallbladder wall of the gallbladder 248 for measuring thicknesses. The candidate regions are identified according to steps of a physics based model executed by the processing unit 220, rather than a data driven machine learning model.

The candidate regions module 233 begins by extracting intermediate feature maps from the machine learning model implemented by the location modeling module 232. The machine learning model generates the intermediate feature maps for estimating the thickness measurement location of the set of calipers, as discussed above. The intermediate feature maps may be extracted anytime, i.e., before, during, or after inference. The intermediate feature maps show different portions of the gallbladder wall, respectively, as highlighted salient regions. As discussed above, the intermediate feature maps are extracted from one of the end layers of the trained machine learning model.

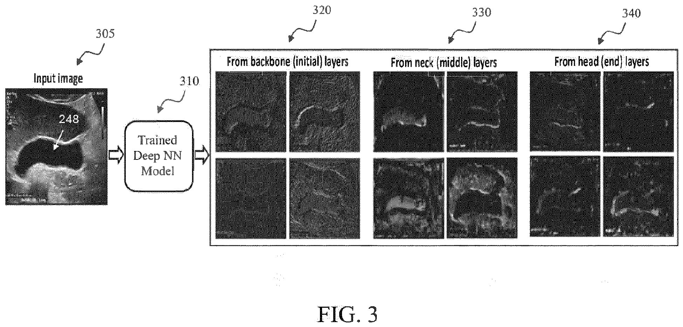

The Fig. 3 shows illustrative images showing feature maps at different layers generated by the machine learning model (e.g., deep neural network implemented by YOLO) based on a sagittal view of a gallbladder, according to a representative embodiment. Referring to Fig. 3, an ultrasound image 305 of the gallbladder 248 is input to the trained YOLO deep neural network model 310. For purposes of illustration, the ultrasound image 305 is the sagittal view of the gallbladder 248, although it is understood that the transverse view of the gallbladder 248 may be used as well. Four illustrative sets of feature maps are shown extracted from different layers of the architecture, including initial (backbone) layers 320, middle (neck) layer 330, and end (head) layers 340. By interpreting the feature maps and the learning pattern of the YOLO deep neural network model 310, it is observed that the initial layers 320 learn to extract the low-level cues from the input ultrasound image 305, which provide basic understanding about the structure and boundaries within the ultrasound image 305. The middle layers 330 appear to start inferring task-specific context, where learning gallbladder wall information is the given task. Hence, the middle layers 330 are observed to focus on anatomy-specific details. The end layers 340 learn to extract high-level cues involving more complex interpretations, such as inferring context and understanding scenes. Since the gallbladder wall of the gallbladder 248 is the target, the end layers 340 are observed to highlight portions of the gallbladder wall, projecting them predominantly. Therefore, the end layers 340 of the trained YOLO deep neural network model 310 provide intermediate feature maps highlighting the gallbladder wall portions, appearing as bright or salient regions, mentioned above.

Accordingly, the intermediate feature maps are extracted from an end layer selected from the end layers 340. The selected end layer is the end layer that activates the most with regard to the number of highlighted salient regions of the gallbladder wall and the best contrast or brightness in the corresponding intermediate feature maps. The end layer that activates the most is consistently the same layer for any input image, and may be determined empirically for selection as the selected end layer. The intermediate feature maps may be extracted by tapping the selected end layer through an application programming interface (API) such that the activation maps of the selected end layer are the intermediate feature maps.

Next, the extracted intermediate feature maps are linearly combined to provide a combined feature map. The combined feature map includes the highlighted salient regions of the gallbladder wall from all of the intermediate feature maps in a single feature map. Linearly combining the intermediate feature maps may be performed by the processing unit 220.

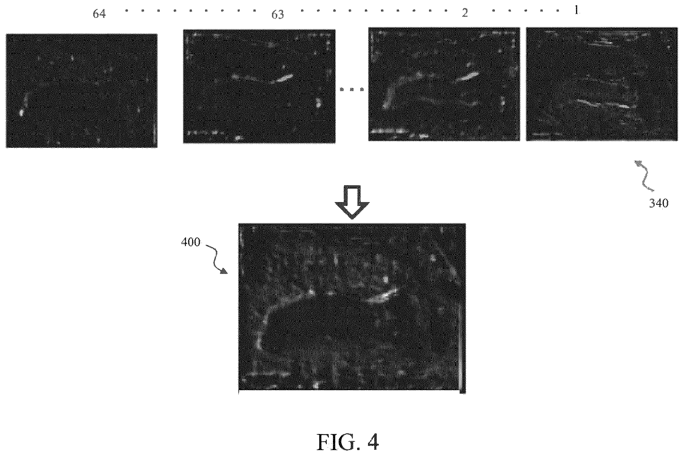

Fig. 4 shows a sampling of the intermediate feature maps extracted from one of the end layers 340 linearly combined into a combined feature map, according to a representative embodiment. Referring to Fig. 4, illustrative 64 feature maps (indicated by 1, 2 ... 63, 64) have been extracted from a selected one of the end layers 340 shown in Fig. 3. The different feature maps 1-64 highlight different portions of the gallbladder wall as salient regions, respectively. The feature maps 1-64 are linearly combined to bring the corresponding salient portions of the gallbladder wall into a single combined feature map 400. The linearly combined feature map 400 shows various portions of gallbladder wall that are suitable for measurements. Here, the combined feature map 400 significantly highlights the anterior portion of the gallbladder wall, which is consistent with clinical guidelines since thickness measurements typically are not done on the posterior wall.

Once the combined feature map 400 is created, adaptive intensity thresholding is performed on pixel intensities of pixels in the combined feature map 400. The adaptive intensity thresholding applies a threshold that preserves the highlighted salient regions of the gallbladder wall in the combined feature map 400. As a result, the adaptive intensity thresholding identifies multiple candidate regions of the gallbladder wall suitable for thickness measurements. That is, the multiple candidate regions correspond to the multiple highlighted salient regions containing pixels with intensity values exceeding the threshold. The pixels having intensity values that do not exceed the threshold are blacked out, visually isolating the candidate regions of interest.

Unlike simple global thresholding, the threshold applied in the adaptive intensity thresholding is determined by statistically examining local regions of the combined feature map 400, and iteratively determining optimal local threshold values for the local regions, respectively. The optimal local threshold values are combined into a global threshold value applied to the entire combined feature map 400. Each of the local threshold values may be determined based on the local image pixel intensity statistics and distribution, and the global threshold value is computed from the local threshold values, as would be apparent to one skilled in the art.

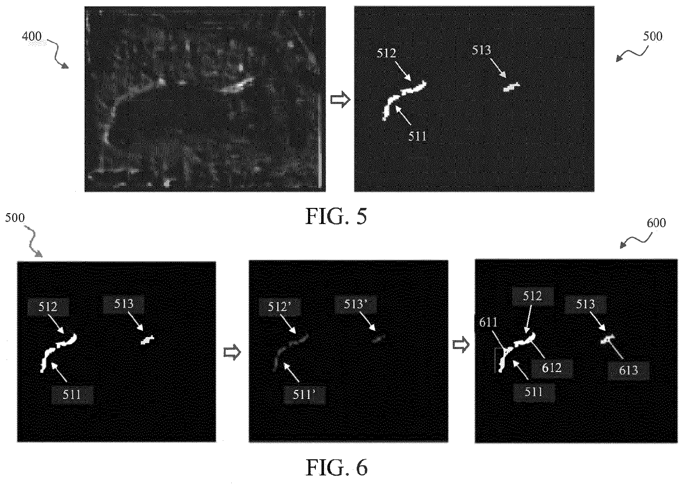

Fig. 5 shows results of adaptive dynamic thresholding performed on the combined feature map providing candidate regions, according to a representative embodiment. Referring to Fig. 5, the adaptive dynamic thresholding is performed on the combined feature map 400. In the depicted example, the adaptive dynamic thresholding results in three different gallbladder wall portions being candidate regions suitable for wall thickness measurements, indicated as first candidate region 511, second candidate region 512, and third candidate region 513 in thresholding feature map 500. The first, second and third candidate regions 511, 512 and 513 correspond to the three most highlighted salient regions (i.e., exceeding the threshold) in the combined feature map 400. Of course, the adaptive dynamic thresholding may provide more or fewer gallbladder wall portions as candidate regions, depending on such factors as the contents of the extracted intermediate feature maps and the value of the threshold used for the adaptive dynamic thresholding, without departing from the scope of the present teachings. The number of possible candidate regions also depends on the clear visibility of the wall boundaries. The combined feature map 400 and the thresholding feature map 500 may be displayed on the display 224.

Referring again to the memory 230 in Fig. 2, caliper placement module 234 is configured to automatically determine locations of sets of calipers on the wall of the gallbladder 248 in the candidate regions for measuring thicknesses of the gallbladder wall. The locations are determined according to steps of a physics based model executed by the processing unit 220, rather than a data driven machine learning model.

The caliper placement module 234 performs skeletonization on the candidate regions obtained by the adaptive dynamic thresholding of the candidate regions module 233 to identify the location of the thickest part of the gallbladder wall in each of the candidate regions. The skeletonization is performed on each candidate region of the candidate regions identified by the adaptive thresholding to determine the thickest part of the gallbladder wall in that candidate region. The gallbladder wall thickness measurement would be performed at the determined thickest part.

Fig. 6 shows the skeletonization process performed on the candidate regions identified in the thresholding feature map to determine thickest parts of the candidate regions, according to a representative embodiment. Referring to Fig. 6, skeletonization may be performed on each of the first, second and third candidate regions 511, 512 and 513 in the thresholding feature map 500, resulting in skeletonized first, second and third candidate regions 511', 512' and 513' showing associated greyscale values. Skeletonization is a morphing technique that reduces binary objects to one-pixel wide representations, which is useful for feature extraction and topology representation, as would be apparent to one skilled in the art. Skeletonization involves calculating the medial axis of a shape, which is the locus of points equidistant from the nearest points on the shape's boundary, and may be referred to as medial axis transform based skeletonization.

Using the greyscale values, the thickest part of each of the first, second and third candidate regions 511, 512 and 513 is determined, as shown in skeletonized feature map 600. That is, the greyscale values are proportional to distances of the pixels from the closest background pixel, respectively. Therefore, the pixel location with the maximum greyscale value is the thickest part of the corresponding candidate region. The skeletonized feature map 600 shows black dots at first, second and third measurement locations 611, 612 and 613 in the first, second and third candidate regions 511, 512 and 513, respectively, representing the thickness computations. The skeletonized feature map 600 may be displayed on the display 224.

Once the locations of the thickest parts in the candidate regions are identified, orientations of the candidate regions themselves are determined. The respective orientation of each candidate region generally corresponds to the longest dimension of that candidate region. The orientation therefore may be determined using ellipse-fitting, for example, according to which the major axis of an ellipse is sized and fitted to the geometric shape of the candidate region to provide the orientation of the candidate region corresponding to the orientation of the ellipse. The orientation at which calipers would be launched for each of the candidate regions is perpendicular to the ellipse's orientation, as discussed below.

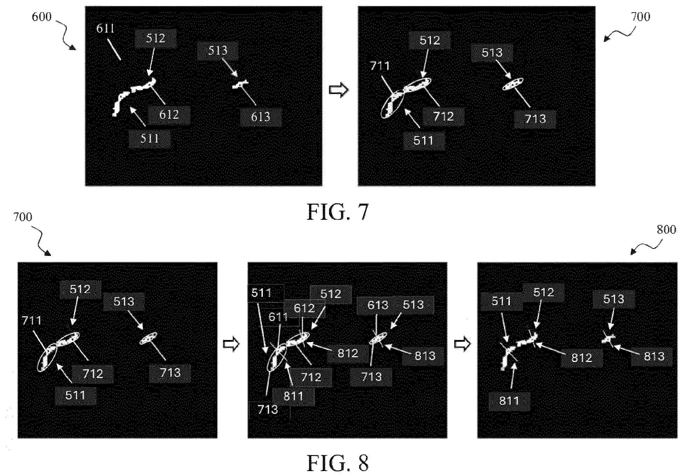

Fig. 7 shows an ellipse-fitting process performed on the candidate regions identified in the skeletonized feature map to determine orientations of the candidate regions, according to a representative embodiment. Referring to Fig. 7, ellipse-fitting may be performed on each of the first, second and third candidate regions 511, 512 and 513 in the skeletonized feature map 600, resulting in first, second and third ellipses 711, 712 and 713 overlapping the first, second and third candidate regions 511, 512 and 513, respectively. Ellipse-fitting is a geometric parameter estimation technique used to approximate the shape of an object or region in a binary image using an ellipse, where the ellipse is sized and oriented with contours of an object (e.g., the candidate regions), as would be apparent to one skilled in the art. Ellipse fitting is particularly useful for an object that is roughly elliptical in shape, allowing for more compact representation and analysis of the object's geometry. As shown, the major axis of the fitted first, second and third ellipses 711, 712 and 713 generally follows the lengthwise dimension of the first, second and third candidate regions 511, 512 and 513, respectively. The orientations of the first, second and third candidate regions 511, 512 and 513 can therefore be approximated by the known orientations of the first, second and third ellipses 711, 712 and 713, shown in ellipse-fitted feature map 700, which helps in launching calipers perpendicular to the gallbladder wall at the thickest parts, indicated by the first, second and third measurement locations 611, 612 and 613 in Fig. 6. The ellipse-fitted feature map 700 may be displayed on the display 224.

Next, caliper placements of a set of calipers on the wall boundaries for each of the candidate regions is determined using the ellipse fitted to the candidate region. Generally, a line is drawn perpendicular to the major axis of the ellipse at the previously determined thickest part of the gallbladder wall in the candidate region. The caliper locations (key points) of the set of calipers on outer and inner wall boundaries are evaluated by the overlapping pixel coordinate information of the perpendicular line with the candidate region.

Fig. 8 shows a process of determining caliper placements in the candidate regions identified in the skeletonized feature map, according to a representative embodiment. Referring to Fig. 8, the first, second and third ellipses 711, 712 and 713 are provided in the ellipse-fitted feature map 700. The first, second and third measurement locations 611, 612 and 613 indicating the thickest parts of the corresponding first, second and third candidate regions 511, 512 and 513 are also provided. First, second and third caliper placement lines 811, 812 and 813 are drawn through the first, second and third measurement locations 611, 612 and 613 perpendicular to the major axes of the first, second and third ellipses 711, 712 and 713, respectively. The locations at which each of the first, second and third caliper placement lines 811, 812 and 813 intersect the outer and inner wall boundaries of the gallbladder wall correspond to the key points at which the calipers in a set of calipers are to be placed for the corresponding first, second and third candidate regions 511, 512 and 513, respectively. The first, second and third caliper placement lines 811, 812 and 813 are shown with the corresponding first, second and third candidate regions 511, 512 and 513 in caliper placement feature map 800. The caliper placement feature map 800 may be displayed on the display 224.

Sets of calipers are then launched in the different candidate regions on the gall bladder walls in the ultrasound image to provide options for caliper placements to measure gallbladder wall thickness. Each set of calipers defines a measurement location for gallbladder wall thickness in the corresponding candidate region.

One of the candidate regions is selected as the preferable measurement location based on information provided by the launched sets of calipers. The selection may be based on various criteria, such as the thickest measurement location and/or the measurement location closest to the face of the ultrasound transducer(s) (closest to the top edge of the image), as indicated by the launched sets of calipers. The decision regarding the selection of the candidate region may be performed manually by the user e.g., further based on perception and experience, or automatically by the processing unit 220, e.g., based on predetermined criteria encompassing one or more of the various criteria as mentioned above.

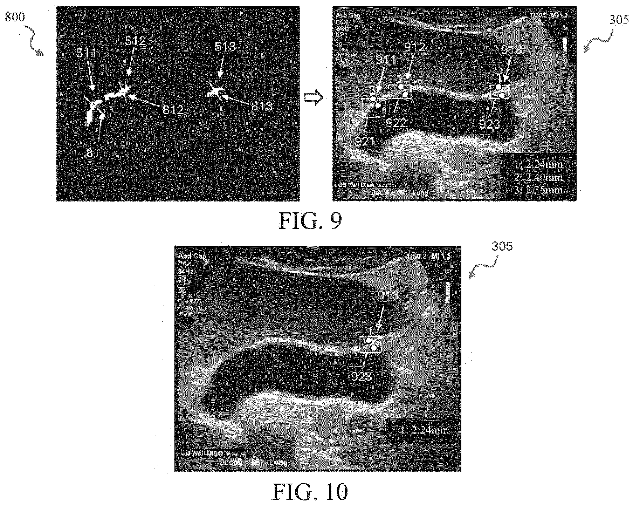

Fig. 9 shows placements of launched calipers in the caliper placement feature map 800 and displayed in ultrasound image 305, according to a representative embodiment. Referring to Fig. 9, the corresponding first, second and third candidate regions 511, 512 and 513 are provided with the first, second and third caliper placement lines 811, 812 and 813 in the caliper placement feature map 800. The first, second and third caliper placement lines 811, 812 and 813 are converted to sets of calipers launched in the original ultrasound image 305 of the gallbladder 248 at their respective locations. That is, the first caliper placement line 811 is converted to a first set of calipers 911, the second caliper placement line 812 is converted to a second set of calipers 912, and the third caliper placement line 813 is converted to a third set of calipers 913. Each set of calipers includes key points on the outer wall boundary and the inner wall boundary of the gallbladder wall at the respective locations of the calipers in the set of calipers. The distance between the key points (calipers) in each set of calipers indicates the thickness of the gallbladder wall at that location.

First, second and third bounding boxes 921, 922 and 923 are generated around the first, second and third sets of calipers 911, 912 and 913 on the ultrasound image 305. The first, second and third bounding boxes 921, 922 and 923 are fit to the dimensions of the first, second and third candidate regions 511, 512 and 513, respectively, as would be apparent to one skilled in the art. The first, second and third bounding boxes 921, 922 and 923 correspond to the first, second and third candidate regions 511, 512 and 513, respectively. The ultrasound image 305, the first, second and third sets of calipers 911, 912 and 913 and the first, second and third bounding boxes 921, 922 and 923 may be displayed on the display 224.

In the depicted example, each of first, second and third bounding boxes 921, 922 and 923 is ordered (1 through 3) based on closeness of the corresponding first, second and third candidate regions 511, 512 and 513 to the transducers of the ultrasound probe 245 (transducer face) at the top of the ultrasound image 305. Accordingly, the first bounding box 921 (closest to the top of the image) is numbered "1," the second bounding box 922 is labeled "2" and the third bounding box 923 (furthest from the top) is labeled "3." Pixel spacing between the calipers in each of the first, second and third sets of calipers 911, 912 and 913 is determined in each of the first, second and third candidate regions 511, 512 and 513. The pixel spacing may be determined using Euclidean distance metric, for example, since the two-dimensional locations of the calipers in the first, second and third sets of calipers 911, 912 and 913 are already known.

Once the pixel spacing is known, the wall thickness of the gallbladder wall in each of the first, second and third candidate regions 511, 512 and 513 is calculated, for example, using Digital Imaging and Communications in Medicine (DICOM) metadata. In the depicted example, the calculated distance between the caliper key points (on opposing wall boundaries) in the first set of calipers 911 is 2.24 mm, the calculated distance between the caliper key points in the second set of calipers 912 is 2.40 mm, and the calculated distance between the caliper key points in the third set of calipers 913 is 2.35 mm. Notably, since the thickness value of the gallbladder wall is less than 3.0 mm at all the three locations, the illustrative gallbladder 248 is identified to be normal.

As mentioned above, one of the first, second or third set of calipers 911, 912 or 913 is selected for measuring the thickness of the gallbladder wall at the respective measurement location. Fig. 10 shows the selected caliper placements displayed in the ultrasound image, according to a representative embodiment. As shown, the first set of calipers 911 has been selected for the caliper placements, and therefore is used for the final wall thickness measurement. The first set of calipers 911 may be selected based on indicating the thickest measurement and/or being closest to the ultrasound probe 245, for example.

The user also may be able to select one of the first, second or third set of calipers 911, 912 or 913 by clicking on the corresponding first, second or third bounding box 921, 922 or 923 via the GUI 128. In addition, or alternatively, the first, second and third sets of calipers 911, 912 and 913 may be associated with specific interface actions to enable selection, such as key board button presses on the user interface 222 and/or displayed button selections on the GUI 228, for example. The selected one of the first, second or third set of calipers 911, 912 or 913 may be identified on the display 224. For example, the corresponding first, second or third bounding box 921, 922 or 923 may be highlighted using a greater brightness or a different color, for example. Also, the unselected sets of calipers may be removed from the display 224, as shown in the ultrasound image 305 in Fig. 10, for example. The thickness measurement corresponding to the selected one of the first, second or third set of calipers 911, 912 or 913 may be displayed as well, as shown in the bottom right corner of the ultrasound image 305 in Fig. 10, for example.

The memory 230 may optionally include a results analysis module (not shown) configured to provide comparisons of the first, second or third set of calipers 911, 912 or 913 for measuring the gallbladder wall, including identifying advantages and/or disadvantages of each. The results analysis module also may provide a recommendation with regard to which of the first, second or third set of calipers 911, 912 or 913 based on various factors, such as proximity to the ultrasound probe 245 and/or quality of the corresponding portion of the gallbladder wall, for example.

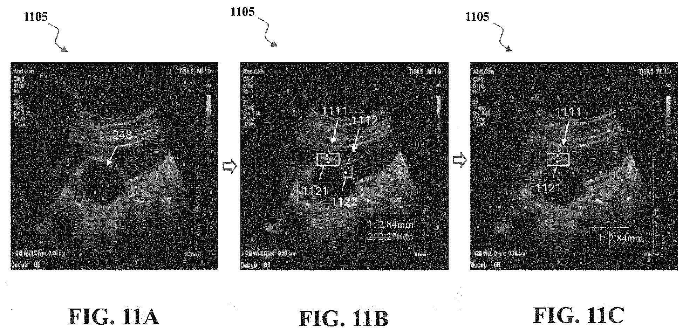

As previously mentioned, the process described above with reference to Figs. 3-10, which is based on a sagittal view of the gallbladder 248, may likewise be implemented based on a transverse view of the gallbladder 248 without departing from the scope of the present teachings. In this regard, Fig. 11A shows an illustrative ultrasound image of a transverse view of a gallbladder for determining caliper placements, Fig. 11B shows caliper placements on the gallbladder wall displayed in the ultrasound image, and Fig. 11C shows selected caliper placements on the gallbladder wall displayed in the ultrasound image, according to a representative embodiment.

Referring to Fig. 11A, ultrasound image 1105 shows a transverse view of the gallbladder 248. Fig. 11B shows caliper placements displayed in the ultrasound image 1105, where the caliper placements have been previously identified in a caliper placement feature map determined as discussed above with regard to the sagittal view. In the depicted example, the caliper placements include a first set of calipers 1111 in a first bounding box 1121, and a second set of calipers 1112 in a second bounding box 1122. The first set of calipers 1111 correspond to a first candidate region, and the second set of calipers 1112 correspond to a second candidate region, which have been identified using adaptive dynamic thresholding performed on a combined feature map, as discussed above. The distance between the calipers in each set indicates the thickness of the gallbladder wall at that location.

The first and second bounding boxes 1121 and 1122 are ordered, e.g., based on closeness of the corresponding first and second candidate regions to the transducers of the ultrasound probe 245 at the top of the ultrasound image 1105. Accordingly, the first bounding box 1121 (closest to the top of the image) is numbered "1" and the second bounding box 922 (furthest from the top) is labeled "2." Pixel spacing between the calipers in each of the first and second sets of calipers 1111 and 1112 is determined in each of the first and second candidate regions. The wall thickness of the gallbladder wall in each of the first and second candidate regions is calculated. In the depicted example, the calculated distance between the key points (calipers) on opposing wall boundaries of the first set of calipers 1111 is 2.84 mm, and the calculated distance between the key points of the second set of calipers 1112 is 2.27 mm. Referring to Fig. 11C, based on the locations and/or thickness values, the first set of calipers 1111 is selected for measuring the thickness of the gallbladder wall at the respective caliper locations. The first set of calipers 1111 is therefore is launched for performing the final wall thickness measurement.

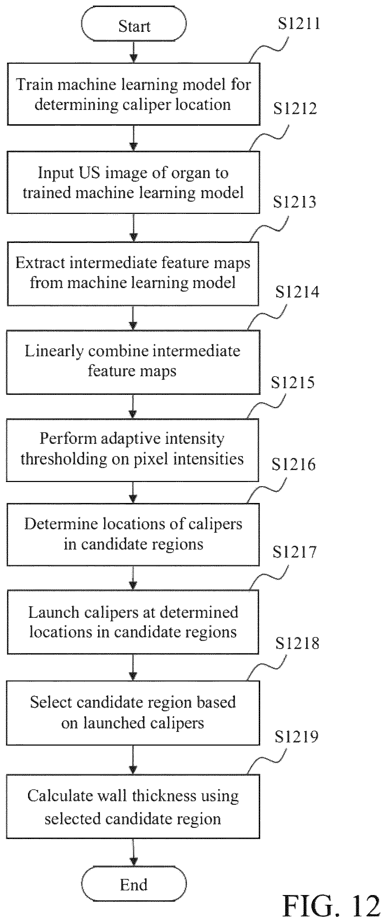

Fig. 12 is a flow diagram of a method of determining caliper placements for measuring wall thickness of a gallbladder, according to a representative embodiment. The method may be implemented at least in part using instructions stored in memory 230 and executable by the processing unit 220 in the system 200, for example. Although the method of Fig. 12 is described with reference to determining caliper placements for measuring wall thickness of a gallbladder, it is understood that the method may apply equally to any organ in the body of a subject that requires wall thickness measurement, such as the uterus, the stomach and the bladder of the subject, for example, as discussed above.

Referring to Fig. 12, the method includes initially training a machine learning model in block S1211 for identifying a location of a set of catheters to measure a gallbladder wall. The machine learning model is trained using training ultrasound images in a training dataset showing gallbladder anatomies, respectively. Each training ultrasound image is annotated to identify locations of calipers in a set of calipers on a gallbladder wall used for measuring thickness of the gallbladder wall, as discussed above. The training may include initializing the machine learning model with a default set of weights and a default set of hyper-parameters.

In block S1212, an ultrasound image of a gallbladder is input to the trained machine learning model. The trained machine learning model is configured to identify a thickness measurement location for placing a set of calipers on a gallbladder wall of the gallbladder that is most suitable for measuring gallbladder wall thickness.

In block S1213, intermediate feature maps are extracted from the machine learning model before, during or after inference of the machine learning model for estimating the thickness measurement location for placing a set of calipers. The intermediate feature maps show different portions of the gallbladder wall, respectively, as highlighted salient regions.

In block S1214, the extracted intermediate feature maps are linearly combined to provide a combined feature map. The combined feature map includes all of the highlighted salient regions of the gallbladder wall from the intermediate feature maps.

In block S1215, adaptive intensity thresholding is performed on pixel intensities of pixels in the combined feature map to identify candidate regions of the gallbladder wall suitable for thickness measurements. The adaptive intensity thresholding is performed using a threshold that preserves the highlighted salient regions of the gallbladder wall. More particularly, performing the adaptive intensity thresholding includes statistically examining the combined feature map to determine the threshold that preserves the highlighted salient regions of the gallbladder wall. Once the threshold is determined, it is applied to the pixel intensities of the pixels in the combined feature map. The pixels having pixel intensities that exceed the threshold are preserved, such that the preserved pixels correspond to the highlighted salient regions of the combined feature map. These highlighted salient regions are identified as the candidate regions, respectively.

In block S1216, locations of calipers in sets of calipers in the candidate regions are determined. The sets of calipers are used for measuring wall thicknesses of the gallbladder, respectively, at the determined locations. The locations of the calipers in the sets of calipers may be determined by performing skeletonization on each candidate region of the candidate regions to determine a thickest part of the gallbladder wall in the candidate region, and performing ellipse-fitting to each skeletonized candidate region. The locations of calipers in a set of calipers corresponding to each candidate region is at the thickest part of the gallbladder wall in the candidate region and has a perpendicular orientation to an orientation of a major axis of the fitted ellipse. In each set of calipers, the individual calipers (key points) are located at the outer and inner boundaries of the gallbladder wall at the thickest part.

In block S1217, calipers in the sets of calipers are launched on the gallbladder wall in each of the candidate regions at the determined locations, respectively. Launching the sets of calipers in the candidate regions may include placing the calipers in each set of calipers on wall boundaries of the gallbladder wall in the corresponding candidate region, where the calipers are connected by a line perpendicular to the gallbladder wall based on the fitted ellipse. The launched sets of calipers may be displayed with the ultrasound image of the gallbladder on a display. The sets of calipers may also be stored and/or output in a report, depending on the user's preference.

In block S1218, a select candidate region of the multiple candidate regions is selected for determining the thickness of the gallbladder wall. The selection of the select candidate region is based on the launched sets of calipers. The selection may be made by the user based on factors such as the user's perception, experience and training. Alternatively, the candidate region may be selected automatically based on predetermined criteria, such as the candidate region with a launched set of calipers defining the thickest location, or the candidate region with a launched set of calipers located closest to the face of the ultrasound transducer(s), for example.

In block S1219, a thickness value of the gallbladder wall is computed at the select candidate region using the launched set of calipers. The thickness value may be computed automatically, e.g., by the processing unit 220. The thickness value may be computed by determining pixel spacing between the calipers in the launched set of calipers, and associating the pixel spacing with a distance, e.g., in millimeters. The thickness value may also be stored and/or output in a report, depending on the user's preferences.

According to the various embodiments, the user is offered multiple gallbladder wall portions for caliper placements, thereby accommodating various user approaches and styles. Also, when a user is dissatisfied with the positioning of a particular set of calipers, alternative candidate regions are available. This avoids the need to manually take another measurement, which may be time-consuming. The embodiments consider the user's expertise, knowledge and perception, and further address a broader market extending beyond geographical limitations, while adhering to clinical guidelines.

In an embodiment, determining caliper placements for measuring wall thickness of a gallbladder may be made adaptable to a specific set of users by learning based on measurement styles and perceptions of the users as a form of feedback for better user adaptability. Since the end decision making process of launching the set of calipers corresponding to the selected candidate region involves user interaction, the determination of which set of calipers to launch may be provided as an active learning system. That is, during an active learning process over time of the machine learning algorithm, discussed above, the machine learning algorithm collects user selections for placement of sets of calipers and provides higher weights to the collected user selections accordingly, thereby learning the style of a specific group of users and eventually adapting to the same. The active learning approach thus enables the intermediate feature maps to learn more efficiently, so that the intermediate feature maps are refined to more predominantly align themselves to the group of users. The selection process thus may be adjusted to deliver desired results customized to the group of users and/or a particular geography, for example, based on understanding corresponding patterns with regard to caliper placements. This embodiment enables the system to evolve based on real-world use and feedback, thereby remain effective and relevant over time.

In another embodiment, other AI models may be adapted for identifying potential gallbladder wall portions for thickness measurements. The machine learning model discussed above may be an AI-based YOLO pose estimation model used to capture feature space representations, which are interpreted to extract candidate regions. However, it is understood that other AI-based YOLO models for object detection, instance/semantic segmentation, classification, and recognition may be incorporated in place of the pose estimation model without departing from the scope of the present teachings, which can be accomplished with additional ground truth annotations beyond the existing key point annotations. Examples of required additional ground truth annotations include boundary contours for segmentation models, multiple bounding boxes for potential wall portions for detection models, outlines of suitable wall portions for recognition models, and sagittal and transverse image classes for classification models. With a deep neural network promptly trained using any of these models, the feature maps from embedded layers may still be relevant for processing and identifying salient regions of the gallbladder wall for thickness computation.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processing unit described herein may be used to support a virtual processing environment.

Although determining caliper placements for measuring wall thickness of a gallbladder or other organs has been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Also, although determining caliper placements for measuring wall thickness of a gallbladder or other organs has been described with reference to particular means, materials and embodiments, it is not intended to be limited to the particulars disclosed; rather determining caliper placements for measuring wall thickness of a gallbladder or other organ extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A method of determining caliper placements for measuring wall thickness of an organ, the method comprising:
inputting an ultrasound image of the organ to a trained machine learning model configured to estimate a thickness measurement location on an organ wall of the organ (S1212);
extracting a plurality of intermediate feature maps from the machine learning model (S1213), wherein the plurality of intermediate feature maps show different portions of the organ wall, respectively, as highlighted salient regions;
linearly combining the extracted plurality of intermediate feature maps to provide a combined feature map including the highlighted salient regions of the organ wall (S1214);
performing adaptive intensity thresholding on pixel intensities of pixels in the combined feature map using a threshold that preserves the highlighted salient regions of the organ wall to identify a plurality of candidate regions of the organ wall suitable for thickness measurements (S1215);
determining locations of calipers in sets of calipers in the plurality of candidate regions for determining wall thicknesses of the organ wall, respectively (S1216);
launching calipers in the sets of calipers on the organ wall in the plurality of candidate regions at the determined locations, respectively (S1217); and
selecting a candidate region of the plurality of candidate regions for determining the wall thickness of the organ wall based on the launched calipers in the sets of calipers (S1218).

2. The method of claim 1, further comprising:
computing a thickness value of the organ wall at the selected candidate region using the launched calipers in the set of calipers (S1219).

3. The method of claim 1 or 2, wherein performing the adaptive intensity thresholding comprises:
statistically examining the combined feature map to determine the threshold that preserves the highlighted salient regions of the organ wall;
applying the threshold to the pixel intensities of the pixels in the combined feature map; and
preserving pixels having pixel intensities exceeding the threshold, wherein the preserved pixels correspond to the highlighted salient regions, which are identified as the plurality of candidate regions, respectively.

4. The method of any of claims 1-3, wherein determining the locations of the calipers in the sets of calipers in the plurality of candidate regions for determining wall thicknesses of the organ comprises:
performing skeletonization on each candidate region of the plurality of candidate regions to determine a thickest part of the organ wall in the candidate region; and
performing ellipse-fitting to each candidate region of the plurality of candidate regions,
wherein locations of calipers in a set of calipers in each candidate region is at the thickest part of the organ wall in the candidate region and has a perpendicular orientation to an orientation of a major axis of the fitted ellipse.

5. The method of claim 4, wherein launching the calipers includes placing the calipers of each set of calipers on wall boundaries of the organ wall connected by a line perpendicular to the organ wall based on the fitted ellipse.

6. The method of any of claims 1-5, further comprising:
initially training the machine learning model using a plurality of training ultrasound images showing organ anatomies, respectively, wherein each training ultrasound image of the plurality of training ultrasound images is annotated to identify locations of calipers in a set of calipers on an organ wall for measuring thickness of the organ wall.

7. The method of claim 6, wherein the training comprises initializing the machine learning model with a default set of weights and a default set of hyper-parameters.

8. The method of any of claims 1-7, wherein the machine learning model is a deep neural network comprising at least one initial layer, at least one middle layer, and at least one end layer, and
wherein the plurality of intermediate feature maps are extracted from the at least one end layer of the deep neural network.

9. The method of claim 8, wherein the deep neural network comprises a You Only Look Once (Yolo), version 8, pose estimation network.

10. The method of claim 9, wherein:
the at least one initial layer extracts low-level cues from the training ultrasound images, and provide basic understanding about structure and boundaries within the input ultrasound image,
the at least one middle layer starts inferring task-specific context regarding anatomy specific details, wherein learning information about the organ walls is a given task, and
the at least one end layer extracts high-level cues involving complex interpretations,
wherein the organ walls are targets causing the at least one end layer to highlight the organ walls by projecting them predominantly.

11. The method of claim 9 or 10, wherein the at least one end layer comprises a plurality of end layers, and wherein the plurality of intermediate feature maps are extracted from a selected end layer of the plurality of end layers that activates the most with regard to the number of the highlighted salient regions of the organ wall and best contrast or brightness in the plurality of intermediate feature maps.

12. The method of any of claims 1-11, further comprising:
displaying the locations of the calipers in the launched sets of calipers as key points within bounding boxes surrounding the sets of calipers in the plurality of candidate regions, wherein the candidate region of the plurality of candidate regions is selected based on the displayed locations of the calipers within the bounding boxes.

13. The method of any of claims 1-12, wherein the organ is a gallbladder.

14. A system for determining caliper placements for measuring wall thickness of an organ, the system comprising:
a display (224);
at least one processing unit (220) coupled to the display; and
at least one non-transitory memory (230) storing instructions which, when executed by the at least one processing unit, cause the at least one processing unit to perform the method of any of claims 1-13.

15. A non-transitory computer readable medium storing instructions which, when executed by at least one processing unit, cause the at least one processing unit to perform the method of any of claims 1-13.
